Europäisches Patentamt

**European Patent Office**

Office européen des brevets

(11) Numéro de publication: **0 055 635**
**A1**

## (12) DEMANDE DE BREVET EUROPEEN

(21) Numéro de dépôt: **81401652.3**

(22) Date de dépôt: **20.10.81**

(51) Int. Cl.³: **A 61 K 9/06**

(30) Priorité: **19.12.80 FR 8027141**

(43) Date de publication de la demande:
**07.07.82 Bulletin 82/27**

(84) Etats contractants désignés:
**BE CH DE GB IT LI LU NL SE**

(71) Demandeur: **Laboratoires du Docteur P. ASTIER**
**42 rue du Docteur Blanche**
**F-75016 Paris(FR)**

(72) Inventeur: **Astier, Patrice**
**Allenmostrasse 118**
**CH-8057 Zürich(CH)**

(74) Mandataire: **Phélip, Bruno et al,**
**c/o Cabinet Harlé & Phélip 21, rue de La Rochefoucauld**
**F-75009 Paris(FR)**

(54) Composition pharmaceutique sous forme d'un gel contenant de l'acide acétylsalicylique.

(57) Gel contenant de l'acide acetylsalicylique comme principe actif.

Ce gel comprend fondamentalement, outre l'acide acétylsalicylique, un solvant permettant une dissolution de l'acide acétylsalicylique sans dégradation et soluble dans l'eau, notamment un solvant glycolique soluble dans l'eau, et un agent gélifiant des milieux non polaires, tel que de préférence un polymère carboxyvinylique.

Applications aux traitements par voie cutanée.

EP 0 055 635 A1

<u>Composition pharmaceutique sous forme d'un gel contenant</u>

<u>de l'acide acétylsalicylique</u>

La présente invention concerne une nouvelle composition pharmaceutique contenant de l'acide acétylsalicylique,et plus précisément un gel contenant de l'acide acétylsalicylique comme principe actif et applicable par voie cutanée.

Il est connu que l'acide acétylsalicylique présente,sur le plan galénique,de nombreux inconvénients qui rendent impossible son utilisaion sous la forme d'une préparation à formulation classique pour application dermique. Les problèmes rencontrés sont de trois ordres essentiellement:problèmes de dissolution ,problèmes de stabilité et problèmes de compatibilité.

L'acide acétylsalicylique est soluble dans l'éther et le chloroforme,dans l'alcool à chaud,mais pas dans l'eau,en règle générale.

L'acide acétylsalicylique est soluble en milieux aqueux alcalin,mais il se produit alors une saponification;il est hydrolysé en acide acétique et en acide salicylique,qui précipite en pH acide.

L'acide acétylsalicylique présente des imcompatibilités avec un certain nombre de principes actifs et d'excipients,tels que les hydroxydes alcalins,les carbonates et les stéarates,entre autres.

La connaissance de ces problèmes et leur prise en compte par l'homme de l'art ont abouti jusqu'à présent à la fabrication de préparations dermiques salicylées ayant pour principe actif des sels de l'acide salicylique. Cependant,ces sels ne présentent pas la même activité thérapeutique que l'acide acétylsalicylique lui-même,dont les propriétés antipyrétiques, analgésiques et surtout anti-in- inflammatoires à effet local sont démontrées depuis longtemps (voir à ce sujet,notamment: The Merck Index; Merck and Co., Inc., Rahway; N.J. USA,9ème édition (1976),ainsi que Martindale: The extrapharmacopeia (27ème édition);édité par Ainleywade,The London Pharmaceutical Press, Londres).

On a maintenant trouvé qu'on peut réaliser une préparation dermique stable renfermant comme principe actif de l'acide acétylsalicylique,et cela en des concentrations qu'on peut choisir à convenance,en utilisant des solvants atopiques particuliers.

L'invention a essentiellement pour objet une composition pharmaceutique sous forme de gel contenant fondamentalement de l'acide acétylsalicylique en tant que principe actif,dans laquelle le gel comprend, outre le principe actif lui-même à la concentration souhaitée,un solvant permettant une dissolution de l'acide acétylsalicylique sans dégradation et soluble dans l'eau,et un agent gélifiant des milieux non polaires.

Selon une forme de réalisation avantageuse, l'objet de l'invention est une composition pharmaceutique sous forme de gel glycolique comprenant fondamentalement,,outre l'acide acétylsalicylique qui en est le principe actif,un solvant glycolique soluble dans l'eau et, comme agent gélifiant,un agent gélifiant des milieux non polaires choisi parmi les polymères vinyliques à groupes carboxyliques,de préférence de qualité pharmaceutique.

Une forme préférée de réalisation d'une composition pharmaceutique selon l'invention comprend un solvant glycolique constitué d'un mélange de propylène glycol et d'un éther glycol,notamment d'éther monoéthylique de diéthylène glycol,un agent gélifiant des milieux non polaires constitué par un polymère carboxyvinylique,tel que par exemple du Carbopol 934(commercialisé sous cette dénomination par B.F. Goodrich),et de l'acide acétylsalicylique en une quantité appropriée pour que le gel formé ait la concentration désirée en ce principe actif.

Une composition pharmaceutique conforme à l'invention tout particulièrement préférée est celle qui a été réalisée au moyen de, pour 100g:

| | |
|---|---|
| Acide acétylsalicylique | 10g environ |
| Ether monoéthylique de diéthy-lène glycol | 40g environ |
| Polymère carboxyvinylique (Carbopol 934) | 3,5 g environ |
| EDTA | 0,05g environ |
| Propylène glycol    q.s.p. | 100g |

La présence dans la composition d'EDTA ou de tout autre agent séquestrant ou stabilisant approprié (qui doit en outre être compatible avec les autres constituants)est préférée,avantageusement dans une proportion de l'ordre de celle qui est indiquée ci-dessus.

La réalisation de ces compositions sous forme de gel est à la portée de l'homme de l'art,qui dispose des connaissances et des techniques qui, éventuellement après quelques tâtonnements,doivent lui permettre d'arriver à un résultat convenable.Dans la pratique,les composants peuvent être ajoutés et mélangés dans l'ordre indiqué dans l'exemple illustratif ci-dessus.

Le gel obtenu a une consistance suffisante pour les applications cutanées et il est lavable à l'eau. Son étalement est satisfaisant,et bien qu'il ait un aspect relativement gras,il n'est pas gras.

Son pouvoir pénétrant est élevé,et cela d'autant plus que le taux d'éther monoéthylique de diéthylène glycol est élevé,au moins dans certaines limites. Sa caractéristique principale est de favoriser la pénétration du principe actif in situ,ce qui lui confère une qualité thérapeutique indiscutable, particulièrement en tant que principe anti-inflammatoire ou antalgique.

Le gel glycolique conforme à l'invention, dont le principe actif unique est l' acide acétylsalicylique,a fait l'objet d'une étude clinique,qui a permis de prouver son activité thérapeutique.

Le gel testé était celui dont la composition est donnée plus haut au titre d'un exemple préféré de composition selon l'invention.

Cette étude a été réalisée sur cinq malades atteints soit de psoriasis,soit d'hyperkératose.

Pour éliminer un éventuel effet placebo,et pour mieux juger de l'efficacité du produit,on a chaque fois pris comme référence un segment de membre atteint,qui ne recevait aucun traitement.

Les doses utilisés ont été variables et fonction de l'étendue des lésions.

Observation 1:

Le malade était un homme de 32 ans,atteint de psoriasis nummulaire des membres, à squames épaisses. Les applications du produit selon l'invention ont été faites le matin,après une toilette au savon neutre. On a observé une détersion complète des lésions traitées en 48 heures,alors que les lésions non traitées étaient inchangées.

Observation 2:

La malade était une femme de 41 ans, atteinte de psoriasis en plaque,très étendu, notamment aux membres inférieurs. Par application du gel selon l'invention,la nuit sous pansement occlusif,on a obtenu en 48 heures une détersion complète,permettant le début d'un traitement aux corticoïdes habituel.

Observation 3

Femme de 35 ans atteinte de psoriasis sur les avants-bras et dans le dos. Par application du gel selon l'invention sous pansement occlusif,on a obtenu une détersion en 24 heures pour les lésions des membres et en 48 heures pour les lésions du dos.

Observations 4 et 5

Un malade atteint d'hyperkératose sur trouble statique de l'avant-pied présentait des lésions habituelles nummulaires sensibles à la pression. Après application du produit selon l'invention,chaque soir,sous pansement occlusif,on a obtenu une détersion dès le troisième jour et le traitement orthopédique a pu être poursuivi.

Tolérance et effets secondaires

Aucun effet secondaire n'a été observé.

La tolérance du gel ayant la composition indiquée plus haut a été excellente dans les cinq cas.

Indications thérapeutiques:

Compte-tenu des résultats susdits et des caractéristiques physico-chimiques du gel à l'acide acétylsalicylique selon l'invention,celui-ci peut être préconisé pour les traitements par voie cutanée des affections suivantes, entre autres:

hyperkératose;

-psoriasis;

-inflammation cutanée;

-contusions avec troubles circulatoires
et trophiques;

-rhumatismes infectieux, rhumatismes
chroniques;

-algies et raideurs musculo-articulaires
post-traumatiques:entorses,hydarthroses,fractures,
blocages articulaires;

-arthrites,arthroses.

Par ailleurs,l'étude toxicologique cutanée
chez le lapin a montré une parfaite tolérance
quotidienne et une bonne pénétration à la dose
indiquée.

L'administration par voie orale du véhicule
a montré également l'absence de toxicité dans le cas
d'ingestions accidentelles.

## REVENDICATIONS

1.Composition pharmaceutique pour applications cutanées comprenant l'acide acétylsalicylique comme principe actif,caractérisée en ce qu'elle constitue un gel comprenant,outre le principe actif, un solvant permettant une dissolution de l'acide a_cétylsalicylique sans dégradation et soluble dans l'eau,et un agent gélifiant des milieux non polaires.

2. Composition selon la revendication 1, caractérisée en ce qu'elle constitue un gel glycolique comprenant fondamentalement,outre l'acide acétylsalicylique qui en est le principe actif,un solvant glycolique soluble dans l'eau et, comme agent gélifiant,un agent gélifiant des milieux non polaires choisi parmi les polymères vinyliques à groupes carboxyliques actifs.

3. Composition selon la revendication 2, caractérisée en ce qu'elle comprend un solvant glycolique constitué d'un mélange de propylène glycol et d'un éther glycol,un agent gélifiant des milieux non polaires constitué par un polymère carboxyvinylique, et de l'acide acétylsalicylique en une quantité appropriée pour que le gel formé ait la concentration désirée en ce principe actif.

4. Composition selon la revendication 3, caractérisée en ce que l'éther glycol est de l'éther monoéthylique de diéthylène glycol.

5.Composition selon la revendication 3,caractérisée en ce qu'elle comprend,pour 100g,environ 10g d'acide acétylsalicylique,environ 40g d'éther monoéthylique de diéthylène glycol,environ 3,5g d'un polymère carboxyvinylique,ainsi que le complément à 100g de propylène glycol.

6.Composition selon l'une quelconque des revendications 1 à 5,caractérisée en ce qu'elle comprend en outre environ 0,05g d'EDTA,pour 100g.

0055635

Office européen
des brevets

**RAPPORT DE RECHERCHE EUROPEENNE**

Numéro de la demande

EP 81 40 1652

| Catégorie | Citation du document avec indication, en cas de besoin, des parties pertinentes | Revendication concernée |
|---|---|---|
| Y | FR - A - 2 110 184 (SYNTEX) <br><br> * page 2, lignes 2-36; page 3, lignes 25-40; page 5, lignes 32 - page 6, ligne 17; page 7, lignes 4-19; revendications 1-3 * | 1-6 |
| Y | FR - A - 2 295 753 (DESPUI) <br><br> * page 1, lignes 1-19; page 1, ligne 38 - page 2, ligne 37; revendications 1-6 * | 1-6 |
| Y | FR - A - 2 345 162 (C.M. INDUSTRIES) <br><br> * page 1, lignes 1-28; exemple 3; revendications 1,2 * | 1-3 |
| Y | US - A - 4 126 681 (PROCTER & GAMBLE) <br><br> * colonne 2, lignes 48-63; colonne 3, ligne 32 - colonne 4, ligne 10; exemple 2 * | 1-3 |
| Y | FR - A - 2 345 161 (C.M. INDUSTRIES) <br><br> * page 1, lignes 1-34; exemple 1 * | 1-3 |
| A | CHEMICAL ABSTRACTS, volume 93, no. 14, 6 octobre 1980, page 339 abrégé 137933w COLUMBUS OHIO (US) ./. | |

**DOCUMENTS CONSIDERES COMME PERTINENTS**

**CLASSEMENT DE LA DEMANDE (Int. Cl. 3)**

A 61 K 9/06

**DOMAINES TECHNIQUES RECHERCHES (Int. Cl. 3)**

A 61 K 9/00

**CATEGORIE DES DOCUMENTS CITES**

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 02-03-1982 | CONTET |

OEB Form 1503.1   06.78

Office européen
des brevets

**RAPPORT DE RECHERCHE EUROPEENNE**

| DOCUMENTS CONSIDERES COMME PERTINENTS | | | CLASSEMENT DE LA DEMANDE (Int. Cl. ³) |
|---|---|---|---|
| Catégorie | Citation du document avec indication, en cas de besoin, des parties pertinentes | Revendication concernée | |
| | & Atti. Soc. Nat. Mat. Modena 1978, 109, 119-30 M.T. BERNABEI et al. "Effect of adjuvants on the solubility of salicylic acid. Correlation with absorption" <br><br> * abrégé en entier * <br><br> ---------- | 1 | |
| | | | **DOMAINES TECHNIQUES RECHERCHES (Int. Cl. ³)** |

**OEB Form 1503.2   06.78**